# EUROPÄISCHE PATENTSCHRIFT

(11) **EP 0 355 299 B1**
(45) Veröffentlichungstag und Bekanntmachung des Hinweises auf die Patenterteilung: **25.05.1994**
(21) Anmeldenummer: 89111021.5
(22) Anmeldetag: 17.06.1989
(51) Int. Cl.: A61H 33/02

(54) **Strömungs-Behandlungsgerät**
Hydrotherapy device
Dispositif d'hydrothérapie

(30) Priorität: 30.06.1988 DE 3822176
(43) Veröffentlichungstag der Anmeldung: 28.02.1990
(73) Patentinhaber: Heddernheimer Metallwarenfabrik GmbH, D-78464 Konstanz (DE)
(72) Erfinder: Baumann, Peter, D-7750 Konstanz (DE)
(74) Vertreter: Patentanwälte Ruff, Beier, Schöndorf und Mütschele

(56) Entgegenhaltungen:
- EP-A- 0 120 354
- FR-A- 2 520 612
- US-A- 3 267 936

## Beschreibung

Die Erfindung betrifft ein Strömungsbehandlungsgerät zur Behandlung des menschlichen Körpers, insbesondere eine Sprudelbadeeinrichtung gemäß dem Oberbegriff des Anspruchs 1.

Mit solchen Geräten werden beispielsweise auf die Haut oder Muskulatur der zu behandelnden Person unterschiedlich starke Reize ausgeübt, um Körperfunktionen, wie Durchblutung, Kreislauf und Stoffwechsel durch unterschiedliche Belastungszustände und durch psychische Entspannung zu fördern. Derartige Reize können mechanische Vibrationen mit Massagegeräten, elektrische Reize mit Reizstromgeräten oder Strömungsbeaufschlagungen mit Luft- bzw. Wasserströmen insbesondere im Badewasser sein.

Die Aufbewahrung der hinsichtlich ihrer Flächenerstreckung meist recht großen und selbst im zusammengefalteten Zustand sperrigen Behandlungseinheit im Nichtgebrauchsfalle bereitet meist Schwierigkeiten.

Die US-A-3 267 936 sie liegt dem Oberbegriff des Anspruchs 1 Zugrunde beschreibt ein Gerät mit einem Ringrohr als Behandlungseinrichtung, die in eine Badewanne eingelegt und über einen Schlauch mit einem Behälter, der ein Gebläse enthält, verbunden werden kann. Zur Aufbewahrung kann die Behandlungseinrichtung im zerlegten Zustand in einen Teil des Behälters eingebracht werden, der einen Raum, in dem sich das Gebläse befindet, annähernd U-förmig umschließt. Dabei werden die aus Rohren gebildeten Teile der Behandlungseinrichtung durch Ausnehmungen eines Zwischenbodens gesteckt und gesichert. Eine Verwendung mattenförmiger Behandlungseinrichtungen ist nicht offenbart.

Durch die DE-A-32 02 862 und die die Priorität dieser deutschen Anmeldung beanspruchende FR-A-2 520 612 ist es bekannt, in einem Aufnahmebehälter der Grundeinheit unterhalb des Gebläses die vollständig zusammengefaltete oder gerollte Behandlungseinheit sowie ggf. den Luftschlauch unterzubringen, wobei die Behandlungseinheit in der Regel von der Seite oder von oben nach Abheben des Gebläses eingesetzt werden muß. Dies ist auch bei dem Luftsprudel-Massagegerät nach der DE-A-33 11 103 (EP-A-0 120 354) der Fall, wo jedoch der Aufnahmebehälter benachbart hinter dem Gebläse vorgesehen ist. In beiden Fällen muß die Behandlungseinheit zu einem Paket voll faltbar ausgebildet sein, wobei im ersten Fall auch eine Unterbringung als Rolle denkbar ist. Sowohl die aufrollbare wie die voll zum Paket faltbare Ausbildung der Behandlungseinheit ist aufwendig, zumal die entsprechenden Falt- oder Rollzustände nur für die Aufbewahrung und nicht für die Anwendung benötigt werden. Außerdem ergibt sich dadurch, daß das gewichtsmäßig verhältnismäßig schwere Gebläse entweder oberhalb oder in Höhe der Oberkante der aufbewahrten Behandlungseinheit bzw. des zugehörigen Aufbewahrungsraumes liegt, eine ungünstig hohe Lage des Schwerpunktes des Grundgerätes, das meist als Standgerät auf dem Badezimmerboden steht und im Interesse der Sicherheitserfordernisse, insbesondere der Vermeidung der Beschädigung von elektrisch leitenden Teilen, eine besonders gute Standsicherheit haben sollte.

Der Erfindung liegt die Aufgabe zugrunde, Nachteile bekannter Ausbildungen zu vermeiden, insbesondere ein Strömungsbehandlungsgerät der genannten Art zu schaffen, welches so konzipiert ist, daß bei raumsparender Bauweise die Ausstattung des Grundgeräts verändert und damit an die jeweiligen Erfordernisse angepaßt werden kann.

Diese Aufgabe wird gelöst durch ein Behandlungsgerät mit den Merkmalen des Anspruchs 1. Bei dem Strömungsbehandlungsgerät mit einer Behandlungseinheit, die über einen Verbindungsstrang anschließbar ist an ein Grundgerät, das ein Gerätegehäuse mit einem Generator, vorzugsweise einem Druckluft-Gebläse, und einen annähernd U-förmigen Aufbewahrungsraum für die Behandlungseinheit aufweist, wobei der U-förmige Aufbewahrungsraum eine der Behandlungseinheit entsprechende Höhe hat und einen weiteren Gehäuseraum teilweise umschließt, in dem der Generator angeordnet ist, ist die Behandlungseinheit eine Körpermatte und das Grundgerät ist aus mindestens zwei Baugruppen zusammengesetzt, wobei jede Baugruppe mindestens eine Funktionseinheit, wie den Generator, eine Rückflußkammer oder Steuer- und Bedienungselemente aufweist. Dadurch braucht auch eine mattenartige Behandlungseinheit weder eng faltbar noch eng rollbar ausgebildet zu werden, sondern es genügen bereits wenige Gelenke oder ein verhältnismäßig großer Rollradius für eine raumsparende Unterbringung in dem Gerätegehäuse bzw. dem Aufbewahrungsraum. Der modulartige Aufbau erleichtert den Austausch von Funktionseinheiten durch einfachen Austausch der entsprechenden Baugruppe.

Bevorzugte Ausführungsformen des Behandlungsgeräts gemäß der Erfindung sind in den Unteransprüchen beschrieben.

Der Aufbewahrungsraum kann im Horizontalschnitt oder im Vertikalschnitt U-förmig nach Art eines, aneinander anschließende Abschnitte aufweisenden Hohlkörpers ausgebildet sein, wobei beide U-Schenkel zweckmäßig zur Rückseite des Grundgerätes ausragen, während sie im Falle der zweiten Ausbildung nach oben ragen können. Im Falle der ersten Ausbildung kann ein gegenüber benachbarten Längsabschnitten um etwa 90° klappbarer Längsabschnitt der Behandlungseinheit im Aufbewahrungsfall an der Rückseite des Grundgerätes liegen und beispielsweise die wenigstens im Bereich des Aufnahmeraumes zur Bildung einer Einschuböffnung offene Rückseite des Gerätegehäuses dann verschließen. Bei einer weiteren Ausführungsform kann die Behandlungseinheit etwa vertikal von der Oberseite des Grundgerätes her in den Aufbewahrungsraum eingesetzt werden. Auch die Möglichkeit, die Behandlungseinheit wahlweise von der Rückseite oder von der Oberseite her einzusetzen, kann vorgesehen sein. Die Entnahme- und Einschuböffnung kann vorzugsweise mit einer gesonderten Abdeckung verschließbar sein.

Der wenigstens teilweise von dem Aufbewahrungsraum umschlossene weitere Gehäuseraum eignet sich insbesondere für die Aufnahme des Generators bzw. des Druckluft-Gebläses derart, daß dieses höchstens bis an die obere und/oder die untere Begrenzung des Aufnahmeraumes reicht und sich somit eine günstig tiefe Lage des Schwerpunktes ergibt.

Bei bekannten Behandlungsgeräten liegt die Hauptachse des Generators, die im Falle eines Druckluft-Gebläses durch die Rotor- bzw. Motorachse gebildet ist, horizontal, was meist zu einer raumaufwendigen Anordnung, einer ungünstigen Schwerpunktlage und im Falle drehender Teile zu ungünstigen Lagerbelastungen führt.

Bei bevorzugten Ausführungsformen der Erfindung ist der Generator mit vertikaler Hauptachse angeordnet. Die Hauptachse verläuft somit im wesentlichen parallel zu den Seitenwänden des Gerätegehäuses. Dadurch kann der Generator in einer an seinen Außenumfang angepaßten Steckmuffe des Gehäuses sicher gehalten, genau zentriert und von oben beispielsweise derart eingesetzt werden, daß er mit mindestens einer nach unten weisenden Stirnfläche auf einer Ringschulter o.dgl. der Steckmuffe sicher ruht. Im Falle eines Gebläses mit baulich vereintem Elektromotor und auf dessen Antriebswelle angeordnetem Gebläserotor liegt der Rotor zweckmäßig unterhalb des Motors, wobei sich unterhalb des Gebläserotors noch ein Auslaßstutzen der Steckmuffe anschließen kann. Steht die Steckmuffe bzw. Halterung für den Generator frei von einer Deckwand des Gehäuses nach unten in das Gerätegehäuse ab, so kann sie in einfacher Weise in der beschriebenen Art von dem Aufbewahrungsraum wenigstens teilweise umschlossen sein.

Um jegliches Einfließen von Wasser in den Generator zu verhindern bzw. eventuell eingedrungenes Wasser sofort abführen zu können, ist bei den beschriebenen Ausführungsformen der Austritt des Generators zweckmäßig an dessen Unterseite oder nach unten gerichtet vorgesehen. Statt dessen oder zusätzlich hierzu kann für den Generator auch eine Rückflußsicherung im Bereich des Austrittes vorgesehen sein, die vorzugsweise ein Rückschlagventil zum Verschließen des Austrittes, einen Naßschalter, wie einen Membranschalter zum Abschalten des Generators o.dgl. ist.

Zusätzlich hierzu oder statt dessen kann eine Rückflußsicherung auch in der Leitungsverbindung zwischen dem Gebläse und der Behandlungseinheit, d.h. der Körpermatte, vorgesehen sein, die zweckmäßig durch einen Auffangspeicher für unerwünscht zurückfließendes Wasser o.dgl. in Form einer Rückflußkammer gebildet sein kann. diese Rückflußkammer ist zweckmäßig durch einen im wesentlichen gegenüber dem übrigen Gerätegehäuse abgedichteten Raum dieses Gehäuses unterhalb des Austrittes des Generators gebildet, in welchen der Generator unmittelbar mündet, so daß im Falle eines Druckluftgebläses in dessen Leitungsverbindung mit der Behandlungseinheit, nämlich zwischen dem Austritt des Gebläses und dem Eingang des Verbindungsstranges ein gegenüber deren Querschnitten wesentlich größerer Druckraum geschaffen ist, in welchem das Gebläse einen Druck aufbaut und aus welchem der den Verbindungsstrang bildende Schlauch versorgt wird. Liegt der Eingang des Schlauches oberhalb des maximalen Speicherpegels der Rückflußkammer, so kann kein dort befindliches Wasser in den Schlauch gedrückt werden; befindet sich dieser Eingang dagegen unterhalb des maximalen Pegels bzw. im tiefsten Bereich der Rückflußkammer, so kann zurückgeflossenes Wasser jederzeit mit dem Generator bzw. mit der von diesem erzeugten Druckluft zurück in die Behandlungseinheit und durch diese in das Badewasser gefördert werden.

Zur Entleerung der Rückflußsicherung kann aber auch in vorteilhafter Weise eine beispielsweise im Boden der Rückflußkammer vorgesehene Entleeröffnung vorgesehen sein, die zur Vermeidung von Druckverlusten zweckmäßig mit einem Deckel, einem manuell betätigbaren Ventil oder mit einem mit einem Steuerkreis betätigbaren Ventil, wie einem Magnetventil, beispielsweise so verschließbar ist, daß das Ablaßventil bei laufendem Generator zwangsgeschlossen und bei stillstehendem Generator zwangsgeöffnet ist. Die Druck- bzw. Rückflußkammer ist zweckmäßig gegenüber dem Stau- bzw. Aufbewahrungsraum für den Behandlungsteil dicht geschlossen. Ist sie gegenüber dem Aufbewahrungsraum dagegen offen, so ist sie vorteilhaft so gestaltet, daß sie gemeinsam mit dem Aufbewahrungsraum einen nach außen abgedichteten bzw. dicht verschließbaren Gesamtraum bildet. In letzterem Fall kann die Rückflußkammer im Nichtgebrauchsfalle wenigstens einen Teil des Aufbewahrungsraumes bilden.

Zur weiteren Vereinfachung der Handhabung des Behandlungsgerätes ist zweckmäßig eine Einzugsvorrichtung zum im wesentlichen vollständig versenkten Einziehen des Verbindungsstranges mit Hilfe einer Antriebskraft, beispielsweise einer über ein Gesperre auslösbaren Federkraft vorgesehen, wobei die Einzugsvorrichtung zweckmäßig eine Haspel, Trommel o.dgl. zum Aufwickeln des Verbindungsstranges bzw. -schlauches aufweist. Der Schlaucheingang kann dabei in der Drehachse der Trommel an dieser bzw. an einer von deren Stirnseiten vorgesehen sein. Ist die Wickelachse für den Verbindungsstrang zur Hauptachse des Generators etwa parallel bzw. sogar annähernd achsgleich, so kann der Verbindungsstrang in der Aufbewahrungsstellung gewickelt den Generator bzw. den diesen aufnehmenden Steckstutzen wenigstens teilweise umgeben, so daß eine noch kompaktere Ausbildung des Grundgerätes möglich ist. Das Wickelpaket des aufbewahrten Verbindungsstranges bzw. die hierfür vorgesehene Trommel kann mit horizontaler, bevorzugt aber vertikaler Achse aber auch benachbart zum Generator, zweckmäßig in einer gesonderten Kammer und vorzugsweise innerhalb der Rückflußkammer vorgesehen sein, so daß der zum Beispiel an der Trommel vorgesehene Eingang für den Verbindungsstrang dadurch in dieser auch als Druckkammer vorgesehenen Kammer liegt. Der Verbindungsstrang ist in diesem Fall zweckmäßig beispielsweise über eine Lippendichtung abgedichtet verschiebbar aus der Druckkammer herausgeführt oder er weist im Bereich seines der Druckkammer zugehörigen Endes eine beispielsweise über seinen Außenumfang vorstehende Ringdichtung auf, die sich beim Herausziehen des Verbindungsstranges von innen dichtend an die Begrenzung der Durchführöffnung anlegt. Durch die Entleeröffnung ist auch eine Belüftungsöffnung gebildet, durch welche der eingezogene Verbindungsstrang sowie die Einzugsvorrichtung und die Rücklaufkammer belüftet und daher getrocknet werden können.

Ähnlich verhält es sich auch mit einer vorteilhaft in dem Gerätegehäuse vorgesehenen Kabeleinzugsvorrichtung für ein Netzanschlußkabel, über welches der Generator, Steuereinrichtungen u.dgl. mit elektrischem Strom versorgt werden. Die Kabeleinzugsvorrichtung ist zweckmäßig in einer gesonderten, abgedichteten Gehäusekammer, z.B. unmittelbar benachbart zur Rückfluß- oder Druckkammer bzw. zur Einzugsvorrichtung für den Verbindungsstrang vorgesehen.

Wie bereits beschrieben, ist bei dem erfindungsgemäßen Behandlungsgerät das Grundgerät aus mindestens zwei, jeweils in einem Gehäuseteil eine Funktionseinheit, wie den Generator, die Rückflußkammer, Steuer- und Bedienelemente o.dgl. aufnehmenden Baugruppen zusammengesetzt, so daß unter Aufrechterhaltung einer oder mehrerer Baugruppen durch Austausch einer oder mehrerer anderer Baugruppen die Ausstattung des Grundgerätes verändert und damit an die jeweiligen Erfordernisse angepaßt werden kann. Beispielsweise können unterschiedliche, jeweils Generatoren mit verschiedener Leistung aufweisende Baugruppen in gegeneinander austauschbaren Gehäuseteilen vorgesehen sein oder es können Gehäuseteile mit und ohne Einzugsvorrichtung für den Verbindungsstrang bzw. Kabeleinzugsvorrichtung bzw. Fernbedienung gegeneinander austauschbar sein.

Dabei ist es vorteilhaft, wenn mindestens zwei oder bei mehr als zwei auch alle Baugruppen in Höhenrichtung aufeinander gesetzt und durch vorzugsweise im wesentlichen horizontale Teilungsebenen voneinander getrennt sind, so daß das Grundgerät unabhängig von der jeweils gewählten Ausstattung stets im wesentlichen die gleichen Grundrißmaße aufweist und sich allenfalls unterschiedlich hohe Grundgeräte ergeben.

Diese und weitere Merkmale von bevorzugten Weiterbildungen der Erfindung gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhaft sowie für sich schutzfähige Ausführungen darstellen können, für die hier Schutz beansprucht wird. Ausführungsbeispiele der Erfindung sind in den Zeichnungen dargestellt und werden im folgenden näher erläutert. In den Zeichnungen zeigen:
- Fig. 1: ein erfindungsgemäßes Behandlungsgerät in wesentlich vereinfachter perspektivischer Darstellung;
- Fig. 2: eine weitere Ausführungsform eines Behandlungsgerätes in teilweise vertikal geschnittener Explosionsdarstellung.

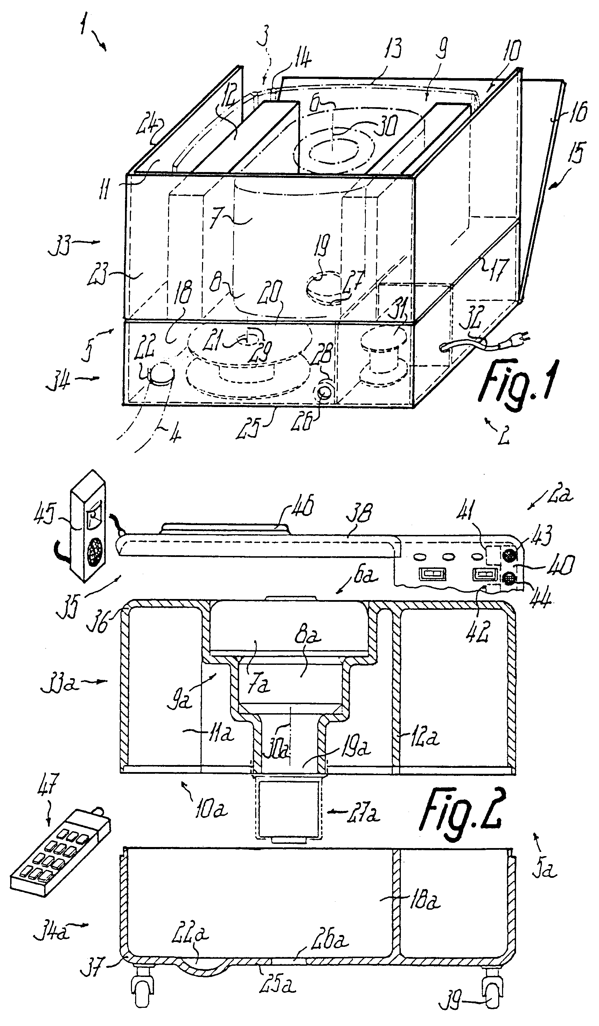

Das in Fig. 1 im wesentlichen im Aufbewahrungszustand dargestellte erfindungsgemäße Behandlungsgerät 1 besteht im wesentlichen aus einem in Fig. 1 an der Oberseite geöffnet gezeigten Grundgerät 2 und einer an bzw. in diesem aufbewahrbaren, strichpunktiert angedeuteten Behandlungseinheit 3 in Form einer matten- bzw. rostartigen Luftverteiler-Unterlage für wenigstens einen Teil der Länge des Körpers der zu behandelnden Person.

An das Grundgerät 2 anschließbar ist ein Verbindungsstrang 4 in Form eines flexiblen Druckluftschlauches, der von dem Grundgerät 2 wegzuführen und an einen Lufteingangs-Anschlußstutzen am Fußende der Behandlungseinheit 3 über eine leicht lösbare Steckverbindung so anzuschließen ist, daß aus über die Länge und Breite der Behandlungseinheit 3 verteilte Luft-Austrittsöffnungen Druckluft zur Seite und/oder nach oben austreten kann. Die Behandlungseinheit 3 ist in der Regel dafür bestimmt, auf dem Wannenboden und/oder an der Rückenfläche einer Badewanne als Unterlage für die badende Person angeordnet zu werden, so daß die austretende Luft in Form von Luftblasen und unter Erzeugung von Wasserströmungen in der gefüllten Badewanne sprudelnd an den Körper der Person gelangt und als Behandlungsmedium wirkt.

Das als Fußboden-Standgerät ausgebildete Grundgerät 2 weist ein gegenüber üblichen Badewannen wesentlich niedrigeres, beispielsweise höchstens etwa 60 cm, vorzugsweise nur etwa 45 cm hohes und in Draufsicht annähernd rechteckig bzw. quadratisch begrenztes Gerätegehäuse 5 auf, dessen Breite und/oder Tiefe in der Größenordnung seiner Höhe liegen kann, so daß es annähernd würfelförmig ist. Das Gerätegehäuse 5 hat annähernd über seine gesamte Höhe im wesentlichen konstante Außenform bzw. Außenquerschnitte. In einer sich über einen Teil der Gehäusehöhe erstreckenden, vorzugsweise annähernd bis zur Gehäuseoberseite reichenden Montageebene befindet sich ein Generator 6 zur Erzeugung des Behandlungsmediums, der im dargestellten Ausführungsbeispiel eine Baueinheit aus einem von einem Elektromotor 7 angetriebenen Gebläse 8 ist, für das Ansaugöffnungen in einer der Außenwandungen des Gehäuses in Form beispielsweise vor nicht näher dargestellten Schlitzen vorgesehen sein können. Der Elektromotor 7 und das Gebläse 8 sind axial aneinander anschließend derart verflanscht, daß das Gebläse 8 an die Unterseite des Elektromotors 7 anschließt und der Gebläserotor unmittelbar auf der Motorwelle angeordnet ist. Für den Generator 6 ist in dem Gerätegehäuse 5 eine Halterung 9 vorgesehen, mit welcher der Generator 6 schwingungsgedämpft lagegesichert ist.

In einer Montageebene des Gerätegehäuses 5, vorzugsweise in derselben Montageebene wie der Generator 6, ist im Gerätegehäuse 5 des weiteren ein Aufbewahrungsraum 10 für die Behandlungseinheit 3 vorgesehen. Der Aufbewahrungsraum 10 ist im dargestellten Ausführungsbeispiel sowohl in Draufsicht wie auch in Vorder- bzw. Rückansicht auf das Gerätegehäuse 5 U-förmig. Der Aufbewahrungsraum 10 ist somit ein in vier Ebenen liegender Hohlraum, von dem die beiden Raumschenkel 11 beiderseits des Generators 6 bzw. der Halterung 9 parallel zueinander liegen und der einen, weiteren Gehäuseraum, nämlich den den Generator 6 aufnehmenden Gehäuseraum umschließt. Die beiden Raumschenkel 11 sind gegenüber diesem weiteren Gehäuseraum bzw. gegenüber dem Generator 6 durch zwei Trennwände 12 abgetrennt, die beiderseits unmittelbar benachbart zum Generator 6 liegen und Bestandteil von dessen Halterung 9 sein können. Die aufrechten Enden dieser Trennwände 12 und/oder ihre Oberseiten sind zweckmäßig jeweils gegenüber der benachbarten Gehäusewand so zurückversetzt, daß die genannten, die Raumschenkel 11 verbindenden Raumteile für die Aufnahme der Behandlungseinheit 3 freibleiben.

Die Höhe des Aufbewahrungsraumes 10, die der Breite der Raumschenkel 11 entspricht, ist nur geringfügig größer als die Breite der Behandlungseinheit 3. Diese besteht zweckmäßig aus zwei oder mehreren, vorzugsweise mindestens drei gelenkig und in ihrer Längsrichtung aneinander anschließenden, platten- bzw. rostartigen Mattenglieder 13 etwa gleicher Grundfläche und Ausbildung, wobei diese Mattenglieder 13 an ihren einander zugekehrten Querkanten über Mattengelenke 14 gelenkig miteinander verbunden sind, deren Gelenkachsen rechtwinklig zur Längsrichtung und etwa in der Ebene der Mattenglieder 13 liegen. Die Behandlungseinheit 3 kann zum Beispiel etwa einen Meter Länge haben und aus vier Mattengliedern von jeweils etwa 25 cm Länge und annähernd 30 cm Breite bestehen. Die Länge mindestens eines, insbesondere eines mittleren Mattengliedes 13, entspricht etwa dem mittleren Abstand zwischen den beiden Raumschenkeln 11 oder einem ganzzahligen Quotienten davon, so daß zum Beispiel im Aufbewahrungszustand zwischen den beiden Raumschenkeln 11 zwei aneinander anschließende Mattenglieder der viergliedrigen Behandlungseinheit 3 liegen können. Mindestens ein äußeres Mattenglied liegt dabei in jedem der beiden Raumschenkel 11, wobei diese äußeren Mattenglieder dann im rechten Winkel zu den mittleren Mattengliedern angeordnet sind. Die einzelnen Mattenglieder 13 brauchen also gegeneinander nur um etwa 90° schwenkbar zu sein.

Gemäß Fig. 1 kann die U-förmig vorgefaltete Behandlungseinheit 13 mit ihren etwa horizontal frei ausragenden Schenkeln voraus von der Rückseite 15 her in den Aufbewahrungsraum 10 eingeschoben werden, bis die Schenkelenden an der gegenüberliegenden Innenseite des Gerätegehäuses 5 und/oder das bzw. die mittleren Mattenglieder an den hinteren Enden der Trennwände 12 anschlagen. Insofern ist an der Rückseite 15 des Gerätegehäuses 5 eine Entnahme- und Einschuböffnung für den Behandlungsteil 3 gebildet. Diese Öffnung bzw. die Rückseite des Gerätegehäuses 5 kann mit einer beispielsweise nach Art einer plattenförmigen Rückwand ausgebildeten Abdeckung 16 verschließbar sein, die entweder nach Art eines Deckels ganz entfernbar oder beispielsweise im unteren Bereich schwenkbar am übrigen Gerätegehäuse 5 angelenkt ist. Die Abdeckung 16 kann in geöffnetem Zustand auch den Generator 6 bzw. dessen Halterung 9 freigeben, so daß dieser für Wartungs- oder Reparaturarbeiten sehr einfach zugänglich ist. Die in der beschriebenen Weise vorgefaltete Behandlungseinheit 3 kann aber auch statt dessen oder zusätzlich hierzu mit nach unten frei ausragenden Schenkeln von oben nach unten in den Aufbewahrungsraum 10 eingesetzt werden, bis die Schenkelenden am Boden des Aufbewahrungsraumes 10 und/oder das bzw. die mittleren Mattenglieder 13 an den Oberseiten der Trennwände 12 aufruhen. In diesem Fall ist an der Oberseite des Gerätegehäuses 5 eine Entnahme- und Einschuböffnung für die Behandlungseinheit 3 gebildet, wobei diese Öffnung ebenfalls mit einer Abdeckung, beispielsweise einem abhebbaren oder aufklappbaren Deckel verschließbar sein kann.

In einer weiteren, gegenüber der zuvor genannten Montageebene wesentlich niedrigeren Montageebene, die durch einen gesonderten Geräte- und Gehäuseteil gebildet ist, sind weitere Funktionseinheiten des Grundgerätes 2 neben- und/oder hintereinander angeordnet. Diese Montageebene ist von der den Aufbewahrungsraum 10 bzw. den Generator 6 aufnehmenden und unmittelbar darüberliegenden Montageebene durch einen etwa horizontalen Zwischenboden 17 im wesentlichen dicht abgetrennt, der den Boden des Aufbewahrungsraumes 10 sowie eine Stützfläche für die Unterseite des Generators 6 bilden kann.

In der unteren, flachen Montageebene bildet das Gerätegehäuse 5 eine nur über einen Teil ihres Grundrisses reichende Druck- bzw. Rückflußkammer 18, die zweckmäßig nur über einen Teil der Gehäusebreite und annähernd über die gesamte Gehäusetiefe reicht und deren Deckwand durch den Zwischenboden 17 gebildet sein kann. In die Rückflußkammer 18 mündet in Form beispielsweise eines Durchbruches im Zwischenboden 17 der Gebläseaustritt 19 und an die Rückflußkammer 18 ist der Schlaucheingang 21 des Verbindungsstranges 4 angeschlossen, so daß dieser über die in der Rückflußkammer 18 als Zwischenkapazität unter Druck stehende Luft versorgt wird.

Innerhalb der Rückflußkammer 18 ist eine schematisch dargestellte Schlaucheinzugsvorrichtung 20 in Form beispielsweise einer unter Federkraft in Aufwickelrichtung ständig belasteten Haspel angeordnet, an deren Kern das innere Ende des Verbindungsstranges 4 angeschlossen sein kann und von welcher der Verbindungsstrang 4 durch eine Schlauchdurchführung 22 im Gerätegehäuse 5 durchziehbar nach außen bzw. zur in der Badewanne liegenden Behandlungseinheit 3 geführt ist.

Das Gerätegehäuse 5 bzw. dessen Gehäuseteil bilden eine im wesentlichen vertikale Vorderwand 23, annähernd rechtwinklig dazu stehende Seitenwände 24 und eine untere annähernd horizontale Bodenwand 25, die im Abstand unterhalb des Zwischenbodens 17 liegt. Die Schlauchdurchführung 22 liegt zweckmäßig im Bereich einer aufrechten Wandung, vorzugsweise im zugehörigen unteren Eckbereich der Vorderwand 23. Die Innenseite der Vorderwand 23 begrenzt vorteilhaft auch die Enden der Raumschenkel 11, während die Seitenwände 24 diese Raumschenkel 11 an ihren voneinander abgekehrten Außenseiten unmittelbar begrenzen. Die Schlauchdurchführung 22 ist so vorgesehen, daß der Verbindungsstrang 4 abgedichtet hindurchgeführt werden kann, daß also trotz der Duchführung die Rückflußkammer 18 nach außen abgedichtet bleibt. Der Schlaucheingang 21 steht in Verbindung mit dem Haspelkern und kann beispielsweise an der oberen oder unteren Stirnseite dieser Haspel vorgesehen sein. Um eventuell in die Rückflußkammer 18 über den Verbindungsstrang 4 eingedrungenes Wasser einfach entfernen zu können, ist die Rückflußkammer 18 mit einer annähernd in ihrem tiefsten Bereich liegenden Entleeröffnung 26 versehen, die in der Bodenwand 25 oder, wie dargestellt, auch in der Vorderwand 23 und zwar zweckmäßig in dem von der Schlauchdurchführung 22 entfernten Eckbereich der Rückflußkammer 18 vorgesehen sein kann.

Der Gebläseaustritt 19 ist mit einem Rückschlagventil 27 gesichert, das zum Beispiel durch ein einfaches Klappenventil gebildet sein kann, welches gegen Federkraft nur durch die den Gebläseaustritt 19 durchströmende Luft geöffnet wird und ansonsten geschlossen bleibt. Die Entleeröffnung 26 ist zweckmäßig mit einem Ablaßventil 28 in Form eines Schaltventils dicht zu verschließen, wobei dieses Ablaßventil 28 zum Beispiel von Hand oder elektromagnetisch geschlossen und geöffnet werden kann. Abschlußventil 28 und Rückschlagventil 27 können miteinander gekoppelt sein.

Die Wickel- bzw. Drehachse 29 der Schlaucheinzugsvorrichtung 20 ist zweckmäßig vertikal bzw. rechtwinklig zum Zwischenboden 17 vorgesehen, wobei dann, wenn die Gebrauchslage des Grundgerätes 2 gegenüber Fig. 1 um 90° verdreht vorgesehen ist und der Zwischenboden 17 eine aufrechte Zwischenwand bildet, die Drehachse 29 ebenso wie die Generatorachse horizontal vorgesehen wäre. Im dargestellten Ausführungsbeispiel ist die Generatorachse bzw. die Hauptachse 30 des Generators 6 und damit des Elektromotors 7 bzw. des Gebläses 8 ebenfalls vertikal vorgesehen, wodurch sich eine sehr raumsparende und einfache Montage ergibt. Die Drehachse 29 kann seitlich gegenüber der Hauptachse 30 versetzt oder im wesentlichen mit dieser fluchtend liegen, so daß zum Beispiel der Gebläseaustritt 19 mit dem Schlaucheingang 21 ebenfalls fluchtet, jedoch zweckmäßig im Abstand gegenüber diesem liegt.

In der zum Aufbewahrungsraum 10 bzw. zum Generator 6 benachbarten Montageebene ist des weiteren in einer gesonderten, insbesondere gegenüber der Rückflußkammer 18 abgedichteten Gehäusekammer eine Kabeleinzugsvorrichtung 31 für ein aus dem Gerätegehäuse 5 herausgeführtes Netzkabel 32 vorgesehen, die vorteilhaft ebenfalls eine durch Federkraft angetriebene Wickelvorrichtung für das Netzkabel 32 aufweist, wobei die Drehachse dieser Wickelvorrichtung vertikal bzw. parallel zur Drehachse 29 und/oder zur Hauptachse 30 vorgesehen sein kann. Die Kabeleinzugsvorrichtung 31 befindet sich unmittelbar benachbart zur Rückflußkammer 18, wobei ihre Gehäusekammer durch eine Zwischenwand von der Rückflußkammer 18 getrennt ist, während die Rückflußkammer 18 und die Gehäusekammer der Kabeleinzugsvorrichtung 31 an ihren voneinander abgekehrten Seiten durch die beiden Seitenwände 24 begrenzt sind. Die Gehäusekammer der Kabeleinzugsvorrichtung 31 ist zweckmäßig an ihrer Vorderseite durch die Vorderwand 23 begrenzt, wobei sie sich vorteilhaft ggf. ebenso wie die Rückflußkammer 18 nur über einen Teil der Gehäusetiefe erstreckt, so daß in der zugehörigen Montageebene noch ein oder mehrere Gehäuseräume zum Beispiel als Stauräume frei bleiben, die zweckmäßig von der Rückseite 15 des Gerätegehäuses 5 her zugänglich und mit der Abdeckung 16 verschließbar sind. Das Netzkabel 32 ist vorteilhaft durch die zugehörige Seitenwand 24 des Gerätegehäuses nach außen geführt.

Durch die voneinander abgesetzten und gesonderten Montageebenen ist es möglich, das Grundgerät 2 aus einzelnen, beispielsweise aufeinandergesetzten Baugruppen 33, 34 zusammenzusetzen und dadurch in einfacher Weise zu modifizieren. Zum Beispiel kann die untere Baugruppe 34 wahlweise mit oder ohne Schlaucheinzugsvorrichtung 20 bzw. Kabeleinzugsvorrichtung 31 ausgebildet werden und beide Baugruppen können wahlweise mit der darüberliegenden Baugruppe 33 kombiniert werden.

Im Falle der Ausbildung nach Fig. 2 ist eine weitere, oberste Baugruppe 35 nach Art einer Abdeckung für die Baugruppe 33a vorgesehen, wobei diese weitere Baugruppe 35 weitere Funktionseinheiten, nämlich zum Beispiel insbesondere die Steuerelemente sowie Bedienelemente für das Behandlungsgerät 1 enthält. Im übrigen sind in Fig. 2 für einander entsprechende Teile die gleichen Bezugszeichen wie in Fig. 1, jedoch mit dem Index "a" verwendet.

Jede Baugruppe 33a, 34a, 35 weist einen gesonderten Gehäuseteil 36, 37, 38 auf und durch gegenseitige Befestigung dieser Gehäuseteile sind die einzelnen Baugruppen miteinander zu einem Gesamtgerät zu verbinden. Die unterste Baugruppe 34a ist als Boden-Baugruppe vorgesehen und kann an ihrer Unterseite Standfüße, selbstlenkende Laufrollen 39 o.dgl. aufweisen. Im dargestellten Ausführungsbeispiel ist der Gehäuseteil 37 dieser Boden-Baugruppe 34a nach oben wenigstens bzw. auch im Bereich der Rückflußkammer 18a offen und von dem Gehäuseteil 36 der darüberliegenden Baugruppe 33a nicht durch einen Zwischenboden getrennt, so daß die Rückflußkammer 18a wenigstens teilweise ebenfalls den Aufbewahrungsraum 10a, nämlich beispielsweise einen Raumschenkel 11a bildet. Zu diesem Zweck ist zum Öffnen des Gerätegehäuses 5a der Gehäuseteil 36 bzw. die zugehörige Baugruppe 33a vom darunterliegenden Gehäuseteil 37 nach Art einer Abdeckhaube abhebbar bzw. zur Seite schwenkbar, so daß der Aufbewahrungsraum 10a zugänglich wird. Bei aufgesetzter Baugruppe 33a greifen die Gehäuseteile 36, 37 derart mit ihren aufrechten Wandungsteilen dicht ineinander, daß die Rückflußkammer 18a nach außen abgedichtet ist. Die Halterung 9a für den Generator 6a ist durch eine von der Deckwand des Gehäuseteiles 36 einteilig nach unten ragende und sich nach unten mehrfach abgestuft verjüngende Gehäusemuffe gebildet, in welcher der Generator 6a von oben zentriert eingesetzt ist und deren unteres Ende die Austrittsöffnung 19a bildet. Zur Sicherung der Austrittsöffnung 19a kann ggf. auch ein Schwimmerventil 27a mit in der Rückflußkammer 18a liegendem Schwimmerkörper vorgesehen sein.

Weiter ist eine Impuls-Steuereinrichtung 40 dargestellt, die so ausgebildet ist, daß der Luftdruck der der Behandlungseinheit zugeführten Druckluft im wesentlichen völlig unregelmäßigen Änderungen bzw. Schwankungen unterworfen werden kann. Zu diesem Zweck weist die Steuereinrichtung 40 zweckmäßig eine Einrichtung 41 in Form eines Steuerteiles auf, über welchen die Motordrehzahl des Elektromotors 7a des Generators 6a im wesentlichen stufenlos oder abgestuft zwischen einer Höchstdrehzahl und einer Mindestdrehzahl verändert werden kann, wobei insbesondere die Mindestdrehzahl so groß gewählt ist, daß immer noch so viel Luft aus der Behandlungseinheit austritt, daß kein Badewasser in diese eindringen kann.

Die Impuls-Steuereinrichtung 40 kann ihrerseits von einem Signalgeber 45 in Form beispielsweise einer im Badezimmer an einer Wand aufzuhängenden Lautsprechereinheit beeinflußbar sein, wofür die Steuereinrichtung 40 einen zum Beispiel nach Art eines Mikrofones ausgebildeten Empfänger 43 für die vom Signalgeber 45 kommenden akustischen Signale aufweist, so daß sich eine drahtlose Wirkverbindung zwischen Signalgeber 45 und Empfänger 43 ergibt, obgleich für die Signalübertragung auch eine Kabelverbindung zwischen dem Signalgeber 45 bzw. einem entsprechenden Verstärker eines Abspielgerätes 46 und dem zugehörigen Eingang des Steuergerätes 40 denkbar ist. Dieses Abspielgerät 46 kann ebenfalls Bestandteil des Grundgerätes 2a oder gesondert von diesem vorgesehen sein.

Des weiteren weist die Steuereinrichtung 40 als Steuerteil eine Einrichtung 42 zur Kompensierung der Steuerträgheit zwischen der Signalabgabe und der Luftdruckänderung in der Behandlungseinheit auf, was zum Beispiel dadurch erreicht werden kann, daß die Tonwiedergabe durch den Signalgeber 45 gegenüber der dem jeweiligen Signal zugehörigen Veränderung der Motordrehzahl verzögert wird. Die Steuereinrichtung 40 einschließlich des Empfängers 43 und ggf. eines weiteren Empfängers 44 für ein Fernbedienungsgerät 47 bzw. das Abspielgerät 46 sind zweckmäßig Bestandteil der obersten Baugruppe 35, die auch ein Bedienungstableau mit Schaltern bzw. Kontroll-Leuchten für die einzelnen Funktionen des Behandlungsgerätes aufweisen kann.

Mit dem zweckmäßig mit Infrarot-Wellen arbeitenden Fernbedienungsgerät 47 sind vorteilhaft zumindest alle wesentlichen Funktionen des Behandlungsgerätes schalt- bzw. einflußbar. Solche Funktionen können das Ein- und Ausschalten des Gerätes, eine Erhöhung und Absenkung des Luftdruckes bzw. der je Zeiteinheit geförderten Luftmenge, das Ein- und Ausschalten der Impuls-Steuereinrichtung 40, das Ändern des Impulsniveaus bzw. des Impulsprogrammes, die Änderung der Empfindlichkeit des dem Empfänger 43 zugehörigen Empfangsteiles, das Ein- und Ausschalten des Abspielgerätes 46, der Eil-Vorlauf bzw. Eil-Rücklauf dieses Abspielgerätes 46, der Wechsel des Tonträgers des Abspielgerätes 46, das Erhöhen oder Verringern der Lautstärke des Signalgebers 45 und ähnliche Funktionen sein.

## Patentansprüche

1. Strömungsbehandlungsgerät (1) zur Behandlung des menschlichen Körpers, insbesondere Sprudelbadeeinrichtung, mit einer Behandlungseinheit (3), die über einen Verbindungsstrang (4) anschließbar ist an ein Grundgerät (2, 2a), das ein Gerätegehäuse (5, 5a) mit einem Generator (6, 6a), vorzugsweise einem Druckluft-Gebläse (8, 8a), und einen annähernd U-förmigen Aufbewahrungsraum (10) für die Behandlungseinheit (3) aufweist, wobei der U-förmige Aufbewahrungsraum (10) eine der Behandlungseinheit (3) entsprechende Höhe hat und einen weiteren Gehäuseraum teilweise umschließt, in dem der Generator (6, 6a) angeordnet ist, dadurch gekennzeichnet, daß die Behandlungseinheit (3) eine Körpermatte ist, und daß das Grundgerät (2, 2a) aus mindestens zwei Baugruppen (33a, 34a, 35) zusammengesetzt ist, wobei jede Baugruppe mindestens eine Funktionseinheit, wie den Generator (6, 6a), eine Rückflußkammer (18) oder Steuer- und Bedienungselemente aufweist.

2. Behandlungsgerät nach Anspruch 1, dadurch gekennzeichnet, daß der Aufbewahrungsraum (10) im Horizontalschnitt oder im Vertikalschnitt U-förmig ist.

3. Behandlungsgerät nach Anspruch 1 oder 2, dadurch gekennzeichnet, daß eine vorzugsweise mit einer Abdeckung (16) verschließbare Entnahme- und Einschuböffnung für die Körpermatte (3) an der Rückseite (15) und/oder an der Oberseite des Gerätegehäuses (5) vorgesehen ist.

4. Behandlungsgerät nach einem der vorhergehenden Ansprüche, dadurch gekennzeichnet, daß bei einem in einem Gerätegehäuse (5) angeordneten Generator (6) dessen Hauptachse (30) aufrecht bzw. vertikal vorgesehen ist.

5. Behandlungsgerät nach einem der vorhergehenden Ansprüche, dadurch gekennzeichnet, daß ein Druckluft-Austritt (19) des Gebläses (8) an dessen Unterseite vorgesehen ist.

6. Behandlungsgerät nach einem der vorhergehenden Ansprüche, dadurch gekennzeichnet, daß für das Gebläse (8) eine Rückflußsicherung im Bereich seines Austrittes, insbesondere ein Rückschlagventil (27), ein Naßschalter o.dgl. angeordnet ist.

7. Behandlungsgerät nach einem der vorhergehenden Ansprüche, dadurch gekennzeichnet, daß in der Leitungsverbindung zwischen dem Gebläse (8) und der Körpermatte (3) vorzugsweise unterhalb des Druckluft-Austritts (19) in dem Gerätegehäuse (5) eine Rückflußsicherung, insbesondere eine Rückflußkammer (18) vorgesehen ist.

8. Behandlungsgerät nach Anspruch 7, dadurch gekennzeichnet, daß die Rückflußkammer (18) eine insbesondere mit einem Ablaßventil (28) schließbare und im Bodenbereich vorgesehene Entleeröffnung (26) aufweist, wobei insbesondere das Ablaßventil (28) bei stillstehendem Gebläse (8) zwangsgeöffnet ist.

9. Behandlungsgerät nach einem der vorhergehenden Ansprüche, dadurch gekennzeichnet, daß mindestens zwei Baugruppen (33a, 34a, 35) übereinanderliegen und ihre Gehäuseteile (36, 37, 38) insbesondere im wesentlichen deckungsgleiche Umfangs-Grundrisse haben.

## Claims

1. Flow treatment apparatus (1) for the treatment of the human body, particularly a bubble bath means, having a treatment unit (3), which is connectable by means of a connecting line (4) to a basic unit (2,2a), which has a unit casing (5,5a) with a generator (6,6a), preferably a compressed air blower (8,8a) and an approximately U-shaped storage area (10) for the treatment unit (3), the U-shaped storage area (10) having a height corresponding to the treatment unit (3) and partly surrounds a further casing space, in which is located the generator (6,6a), characterized in that the treatment unit (3) is a body mat and that the basic unit (2,2a) comprises at least two subassemblies (33a,34a,35), each subassembly having at least one functional unit, such as the generator (6,6a), a return flow chamber (18) or control and operating members.

2. Treatment apparatus according to claim 1, characterized in that the storage area (10) is U-shaped in horizontal section or in vertical section.

3. Treatment apparatus according to claim 1 or 2, characterized in that a removal and insertion opening for the body mat (3) and which is preferably closable with a cover (16), is provided on the back (15) and/or on the top of the apparatus casing (5).

4. Treatment apparatus according to any one of the preceding claims, characterized in that in an apparatus casing (5) is provided a generator (6), whose major axis (30) is upright or vertical.

5. Treatment apparatus according to any one of the preceding claims, characterized in that a compressed air outlet (19) for the blower (8) is provided on its underside.

6. Treatment apparatus according to any one of the preceding claims, characterized in that for the blower (8) is provided a return flow preventer in the vicinity of its outlet, particularly a check valve (27), a wet switch or the like.

7. Treatment apparatus according to any one of the preceding claims, characterized in that in the line connection between the blower (8) and the body mat (3) and preferably below the compressed air outlet (19) in the apparatus casing (5) is provided a return flow preventer, particularly a return flow chamber (18).

8. Treatment apparatus according to claim 7, characterized in that the return flow chamber (18) has an emptying opening (26) located in the bottom area and which is in particular closable with a discharge value (28) and in particular the discharge value (28) is forcibly opened when the blower (8) is stationary.

9. Treatment apparatus according to any one of the preceding claims, characterized in that at least two subassemblies (33a, 34a,35) are superimposed and their casing parts (36,37,38) in particular have substantially congruent circumferential layouts.

## Revendications

1. Appareil (1) de traitement par flux pour le corps humain, notamment dispositif à bain bouillonnant, avec une unité de traitement (3) qui peut être raccordée, par l'intermédiaire d'une conduite de raccordement (4), à un appareil de base (2, 2a) qui présente un carter d'appareil (5, 5a) contenant un générateur (6, 6a), de préférence une soufflante d'air comprimé (8, 8a), et un espace de rangement (10) approximativement en forme de U pour l'unité de traitement (3), l'espace de rangement (10) en U possédant une hauteur correspondant à l'unité de traitement (3) et entourant partiellement un autre espace du carter, dans lequel est disposé le générateur (6, 6a), **caractérisé** en ce que l'unité de traitement (3) est un matelas pour un corps humain et en ce que l'appareil de base (2, 2a) est constitué d'au moins deux sous-groupes (33a, 34a, 35), chaque sous-groupe comprenant au moins une unité fonctionnelle, telle que le générateur (6, 6a), une chambre de reflux (18) ou des éléments de commande et de manoeuvre.

2. Appareil de traitement selon la revendication 1, **caractérisé** en ce que l'espace de rangement (10) est en forme de U en coupe horizontale ou en coupe verticale.

3. Appareil de traitement selon la revendication 1 ou 2, **caractérisé** en ce qu'il est prévu, sur la face arrière (15) et/ou sur la face supérieure du carter d'appareil (5), une ouverture destinée à retirer ou introduire le matelas (3) et pouvant de préférence être fermée par un couvercle (16).

4. Appareil de traitement selon l'une quelconque des revendications précédentes, **caractérisé** en ce que, lorsqu'un générateur (6) est disposé dans un carter d'appareil (5), son axe principal (30) est prévu en position debout ou encore vertical.

5. Appareil de traitement selon l'une quelconque des revendications précédentes, **caractérisé** en ce qu'une sortie d'air comprimé (19) pour la soufflante (8) est prévue sur le dessous de cette dernière.

6. Appareil de traitement selon l'une quelconque des revendications précédentes, **caractérisé** en ce qu'une sécurité contre le reflux pour la soufflante (8), notamment un clapet antiretour (27), un interrupteur réagissant à l'humidité ou similaire, est disposé(e) dans la région de la sortie de cette dernière.

7. Appareil de traitement selon l'une quelconque des revendications précédentes, **caractérisé** en ce qu'une sécurité contre le reflux, notamment une chambre de reflux (18), est prévue dans la conduite de liaison entre la soufflante (8) et le matelas (3), de préférence en dessous de la sortie d'air comprimé (19) dans le carter d'appareil (5).

8. Appareil de traitement selon la revendication 7, **caractérisé** en ce que la chambre de reflux (18) présente une ouverture de vidange (26), prévue dans la région du fond et pouvant notamment être fermée par une soupape d'évacuation (28), la soupape d'évacuation (28) étant notamment à ouverture forcée lorsque la soufflante (8) ne fonctionne pas.

9. Appareil de traitement selon l'une quelconque des revendications précédentes, **caractérisé** en ce qu'au moins deux sous-groupes (33a, 34a, 35) sont superposés, et leurs parties de carter (36, 37, 38) possèdent notamment des contours de base essentiellement coïncidents.
